# EUROPEAN PATENT APPLICATION

(11) **EP 4 403 624 A1**
(43) Date of publication of application: **24.07.2024**
(21) Application number: 22870196.7
(22) Date of filing: 06.09.2022
(51) Int. Cl.: C12N 1/20, A61K 8/99, A61Q 7/00, A61K 35/74, A61P 17/14, A23L 33/135, C12R 1/01

(54) **SCALP MICROBIOME COMPLEX AND USE THEREOF FOR IMPROVING HAIR OR SCALP CONDITION**

(30) Priority: 14.09.2021 KR 20210122765
(71) Applicant: Cosmax, Inc., Hwaseong-si, Gyeonggi-do 18622 (KR)
(72) Inventor: BAEK, Chae Yun, Seongnam-si, Gyeonggi-do 13486 (KR); LEE, Dong Geol, Hwaseong-si, Gyeonggi-do 18496 (KR); KYUNG, Seo Yeon, Seoul 07376 (KR); KANG, Seung Hyun, Seoul 06285 (KR); PARK, Myeong Sam, Seoul 04987 (KR)
(74) Representative: Lavoix
(86) International application number: PCT/KR2022/013364
(87) International publication number: WO 2023/043111

(57) **Abstract**

The present invention pertains to: a novel microorganism; a lysate, a culture, and an extract thereof; and a use thereof for improving hair or scalp condition. A novel Paracoccus denitrificans strain according to an aspect has the effects of promoting hair growth, preventing hair loss, and strengthening hair roots, and can thus be variously applied to improving, preventing, or treating hair or scalp condition. In addition, when the strain is used in combination with one or more Staphylococcus epidermidis strains or yeast extracts, a synergistic effect can be exhibited in improving hair or scalp condition.

## Description

### Technical Field

This application claims priority based on Korean Patent Application No. 10-2021-0122765, filed on September 14, 2021, the entire content of which is incorporated herein by reference.

The present disclosure relates to a novel microorganism, a lysate or a culture solution thereof, an extract of the culture solution, and use of the novel microorganism, the lysate or the culture solution thereof, or the extract of the culture solution for improving a hair or scalp condition.

### Background Art

In the human body, hair grows from the hair follicle located on the scalp, continues to repeat the cycles of anagen, catagen, and telogen during a lifetime, and undergoes generation, growth, and shedding (Paus et al., J Invest Dermatol, 113, pp. 523-532, 1999). During the catagen phase of the hair follicle, the hair follicle stops growing, and cell death and reorganization of the extracellular matrix occur. Therefore, the more hair follicle cells in the catagen and telogen phases of the hair follicle increase, alopecia occurs in which hair falls out and does not grow back.

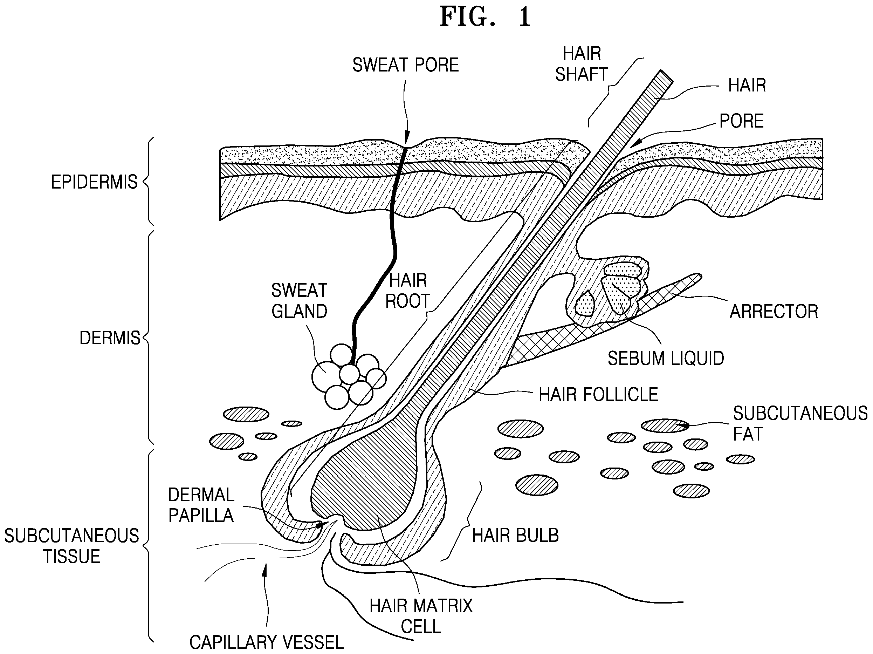

Hair is divided into a visible hair shaft and an invisible hair root, and in the hair root, a root from which hair grows is referred to as a hair bulb, and a lower recessed part of the hair is referred to as a hair papilla (see FIG. 1). A hair papilla is the most important part in hair generation and incudes blood vessels and nerves to thus deliver nutrients and growth factors, thereby promoting hair growth. Since the number of hairs decreases due to the death and growth reduction of dermal papilla cells, the proliferation of dermal papilla cells is necessary to maintain and increase hair.

A vascular endothelial growth factor (VEGF) is a glycoprotein that induces the growth of vascular endothelial cells to promote angiogenesis. A VEGF is attached to VEGF receptor-2 (VEGFR-2) present in a hair papilla to induce a the VEGF-2/ERK pathway and induce the proliferation of dermal papilla cells (Wei Li et al., Exp Cell Res. 2012 Aug 15;318(14):1633-40). In addition, a fibroblast growth factor 7 (FGF7) is also referred to as a keratinocyte growth factor (KGF) and is a proliferation factor that is involved in the growth of an outer part of a hair follicle and a hair part exposed at a scalp surface to adjust a hair production cycle (Genes Dev. 1996;10(2):165-175). Therefore, an increase in expression of VEGF and FGF7 genes in dermal papilla cells may greatly assist in inducing hair growth.

Recently, as environmental pollution and urban stress have become more serious, the number of modern people with alopecia is increasing, and thus interest in materials capable of improving alopecia is increasing. Accordingly, an attempt has been made to invent a material capable of increasing the expression of growth factors in dermal papilla cells of a hair root, which serve to start the production of hair, to induce hair growth to strengthen the hair root and assist in preventing alopecia.

In this regard, Korean Patent No. 1 0-21 02578 discloses a cosmetic composition for promoting hair growth or hair restoration, which includes a plurality of compounds. However, there is not much research on microorganisms capable of improving a hair or scalp condition.

Probiotics are a general term for microorganisms that have beneficial effects on the human body and refer to microorganisms that benefit our bodies. Most probiotics known to date are known as lactic acid bacteria. Probiotics have been reported to have effective efficacy through various beneficial effects on the human body, but research on a correlation between scalp flora and a scalp is insufficient.

Accordingly, there is a need to develop scalp flora usefully usable for scalp-related conditions.

### Disclosure

### Technical Problem

An aspect provides a *Paracoccus denitrificans* strain.

Another aspect provides a lysate or a culture solution of the strain, or an extract of the culture solution.

Another aspect provides a composition including a *P*. *denitrificans* strain, a lysate or a culture solution thereof, or an extract of the culture solution.

Another aspect provides a composition including a combination of: a *P*. *denitrificans* strain, a lysate or a culture solution thereof, or an extract of the culture solution; and a *Staphylococcus epidermidis* strain, a lysate or a culture solution thereof, or an extract of the culture solution.

Another aspect provides a method of preventing, improving, or treating a condition of a subject, the method including treating or administering an effective amount of the composition to a subject in need thereof.

### Technical Solution

An aspect provides a *Paracoccus denitrificans* strain.

The strain may be isolated. As used herein, the term "isolated" refers to a material that does not exist in the natural world but may be artificially isolated and used. The strain may be isolated from a human scalp. The strain may be a strain isolated through a method of purely culturing and isolating a colony cultured by inoculating a sample (for example, a corneocyte) obtained from a human scalp into Reasoner's 2A (R2A) medium. Therefore, the strain may belong to a scalp microbiome.

The strain may be a strain belonging to *Paracoccus sp.*

The strain may include 16S rRNA having a sequence identity of about 95 % or more, about 96 % or more, about 97 % or more, about 98 % or more, about 99 % or more, about 99.5 % or more, or about 99.9 % or more, with SEQ ID NO: 1. The strain may include 16S rRNA of SEQ ID NO: 1.

As used herein, the term "sequence identity" refers to a degree of sameness in an amino acid residue or a base in a specific region of two sequences that are aligned to best match each other for comparison. The sequence identity may be confirmed according to methods known in the art. A percentage of sequence identity may be determined by using a known sequence comparison program, for example, BLASTN (NCBI), CLC Main Workbench (CLC bio), or MegAlign^{™} (DNASTAR Inc).

The *P. denitrificans* strain may be a strain deposited under the deposit number KCCM12994P. The strain deposited under the above deposit number may be a *Paracoccus denitrificans* SC-1 strain.

The strain may have an effect of improving a hair or scalp condition. For example, the improving of the hair or scalp condition may include at least one of hair growth promotion, alopecia prevention, and hair root strengthening.

Another aspect provides a lysate or a culture solution of the strain, or an extract of the culture solution.

Specific details of the strain are as described above.

As used herein, the term "disruption solution" may be used interchangeably with "lysate" and may refer to a product obtained by breaking a cell wall of a strain by using a chemical or physical force. The lysate may include the lysate itself or a concentrate or lyophilisate thereof.

As used herein, the term "culture solution" may be used interchangeably with "culture supernatant," "conditioned culture medium," or "conditioned medium" and may refer to the entire medium including the strain, a metabolite thereof, extra nutrients, and the like, wherein the strain is obtained by culturing the strain for a certain period of time in a medium capable of supplying nutrients such that a *P*. *denitrificans* strain may grow and survive *in vitro.* In addition, the culture solution may refer to a culture solution obtained by removing bacterial cells from a bacterial culture solution obtained by culturing a strain. Meanwhile, a liquid in which bacteria cells have been removed from the culture solution is also referred to as "supernatant," and the supernatant may be obtained by leaving the culture solution for a certain period of time to take only a liquid in an upper layer excluding a precipitated part in a lower layer, by removing bacterial cells through filtration, or by centrifuging the culture solution to remove a precipitate at a lower side and take only a liquid at an upper side. The "bacterial cell" refers to the strain itself of the disclosure and includes a strain itself isolated and selected from a scalp sample or the like or a strain obtained by culturing and isolating the strain from a culture solution. The bacterial cell may be obtained by centrifuging a culture solution to take a portion precipitated in a lower layer or by leaving a culture solution for a certain period of time and then removing a liquid at an upper side because the cell precipitates to the lower layer of the culture solution due to gravity.

The culture solution may include a culture solution itself obtained by culturing a strain, a concentrate or lyophilisate thereof, a culture supernatant obtained by removing the strain from the culture solution, or a concentrate or lyophilisate thereof.

Culture media and culture conditions for cultivating the *P. denitrificans* strain may be appropriately selected or modified by those skilled in the art. For example, the culture solution may be obtained by culturing the *P. denitrificans* strain in a medium (for example, an R2A broth) at any temperature of more than 10 °C or less than 40 °C for a certain period of time, for example, 4 hours to 72 hours.

As used herein, the term "extract of a culture solution" may refer to an extract extracted from the culture solution or a concentrate thereof and may include an extract, a diluted liquid or concentrate of the extract, a dried product obtained by drying the extract, a crude product or purified product thereof, or a fraction obtained by fractionating the same.

The strain, the lysate or the culture solution thereof, or the extract of the culture solution may be biologically pure. The strain may be obtained through pure culture.

Another aspect provides use of a *Paracoccus denitrificans* strain, a lysate or a culture solution of the strain, or an extract of the culture solution. Specifically, a composition including a *Paracoccus denitrificans* strain, a lysate or a culture solution thereof, or an extract of the culture solution is provided.

In the above-described use or composition, the *P. denitrificans* strain may be the *P. denitrificans* strain according to an aspect.

Use of the strain may include improving a hair or scalp condition.

The improving of the hair or scalp condition may include at least one of hair growth promotion, alopecia prevention, hair root strengthening, scalp lipid suppression, dandruff prevention, and scalp inflammation suppression. In an embodiment, the improving of the hair or scalp condition may include at least one of hair growth promotion, alopecia prevention, and hair root strengthening.

As used herein, the term "hair growth" refers to hair production and growth.

As used herein, the term "hair growth promotion" is used interchangeably with "hair production promotion" and means that hair production and growth are promoted to increase a proportion of hair in the anagen to the total hair. The hair growth promotion includes not only promoting the production of new hair, but also keeping the existing hair growing healthily.

As used herein, the term "alopecia" refers to a phenomenon in which hair is removed from a scalp or a state in which hair is sparse or thin. The alopecia may be caused by a decrease in proportion of hair in the anagen. The alopecia may be caused by extrinsic factors or endogenous factors. The extrinsic factors refer to various external factors such as ultraviolet rays, and the endogenous factors are factors that occur over time and include cell death of dermal papilla cells due to telomeres, oxidative stress due to a cellular dysfunction, and the like. The alopecia may include at least one selected from alopecia areata, androgenetic alopecia, telogen effluvium, traumatic alopecia, alopecia due to trichotillomania, pressure alopecia, anagen effluvium, alopecia pityroides, alopecia syphilitica, alopecia seborrhecia, symptomatic alopecia, scarring alopecia, and congenital alopecia, but is not limited thereto.

As used herein, the term "alopecia prevention" means preventing or suppressing alopecia. Accordingly, the alopecia prevention may include alopecia improvement, alopecia prevention, alopecia treatment, and the like.

As used herein, the term "hair root strengthening" refers to any action that makes a hair root of hair strong and healthy. For example, a hair root may be strengthened by proliferation of dermal papilla cells, promotion of nutrient delivery to dermal papilla cells, and increased expression of growth factors in dermal papilla cells.

As used herein, the term "scalp lipid" refers to lipid produced in a scalp. When lipids accumulate excessively on a scalp, it causes problems such as shine, dandruff, stinging, and itching. When sebum is excessively secreted, a lipid film is formed on a scalp. When the lipid film is excessively formed on the scalp, anaerobic bacteria propagate, and fatty acid production increases, which may cause various troubles, seborrheic scalpitis, bad odor, alopecia, and the like. The lipid may refer to peroxidized lipid.

As used herein, the term "scalp lipid suppression" refers to any action that reduces or inhibits the production of lipid on a scalp. Scalp lipid may be suppressed to obtain effects of preventing dandruff, improving or suppressing scalp inflammation and itching caused by sebum, and preventing alopecia caused by lipid peroxide.

As used herein, the term "dandruff" refers to a phenomenon in which epidermis is shed from a scalp, and thus dead skin cells appear prominently. Dandruff may be accompanied by itching.

As used herein, the term "dandruff prevention" means preventing or suppressing the production of dandruff. Therefore, the dandruff prevention may mean improving or preventing dandruff.

As used herein, the term "scalp inflammation" refers to inflammation occurring in a scalp. The scalp inflammation may include at least one of seborrheic dermatitis, pruritus, dry eczema, erythema, urticaria, psoriasis, drug rash, scalp acne, and the like.

As used herein, the term "scalp inflammation suppression" refers to any action that reduces or improves scalp inflammation or prevents or suppresses the occurrence of scalp inflammation.

As used herein, the term "prevention" includes suppressing the occurrence of a disease.

As used herein, the term "treatment" includes suppressing, alleviating, or eliminating the development of a disease.

The composition according to an aspect may include the *Paracoccus denitrificans* strain, the lysate or the culture solution thereof, or the extract of the culture solution to proliferate human follicular dermal papilla cells (HFDPCs). Therefore, the composition may have a hair root strengthening effect, an alopecia prevention effect, and a hair growth promotion effect.

The composition according to an aspect may include the *P. denitrificans* strain, the lysate or the culture solution thereof, or the extract of the culture solution to increase the expression of at least one of a vascular endothelial growth factor (VEGF) and a fibroblast growth factor 7 (FGF7). Due to an increase in expression of a growth factor such as the VEGF or the FGF7, nutrient delivery to dermal papilla cells may be promoted, and growth of hair root cells may be induced, thereby strengthening a hair root, preventing alopecia, and also promoting hair growth. Therefore, the composition may have a hair root strengthening effect, an alopecia prevention effect, and a hair growth promotion effect.

In another embodiment, the strain may be used together with another strain that has an effect of improving a hair or scalp condition, for example, a strain belonging to *Staphylococcus* sp. or *Paracoccus* sp. to have a synergistic effect.

In another embodiment, the composition may further include *Staphylococcus epidermidis,* a lysate or a culture solution thereof, or an extract of the culture solution to have a synergistic effect of improving a hair or scalp condition. Accordingly, the composition includes a combination of the *P. denitrificans* strain, the lysate or the culture solution thereof, or the extract of the culture solution; and the *S*. *epidermidis,* the lysate or the culture solution thereof, or the extract of the culture solution.

The *S*. *epidermidis* may be isolated from a human scalp. The *S*. *epidermidis* may include 16S rRNA having a sequence identity of about 95 % or more, about 96 % or more, about 97 % or more, about 98 % or more, about 99 % more, about 99.5 % or more, or about 99.9 % or more with SEQ ID NO: 2. The *S*. *epidermidis* may include 16S rRNA of SEQ ID NO: 2. The *S*. *epidermidis* may be a strain deposited under the deposit number KCCM12559P. A strain deposited under the deposit number KCCM12559P may be a *Staphylococcus epidermidis* CICARIA strain.

In another embodiment, the composition may further include a yeast extract to have a synergistic effect of improving a hair or scalp condition.

Therefore, (1) the *Paracoccus denitrificans* strain, the lysate or the culture solution thereof, or the extract of the culture solution; and (2) a combination of one or more of the *S. epidermidis,* the lysate or the culture solution thereof, or the extract of the culture solution; and the yeast extract is used interchangeably with the term "scalp microbiome complex" used herein.

For example, the composition may include a combination of: the *P. denitrificans* strain, the lysate or the culture solution thereof, or the extract of the culture solution; the *S. epidermidis,* the lysate or the culture solution, or the extract of the culture solution; and the yeast extract.

The composition may include a strain, a lysate or a culture solution thereof, or an extract of the culture solution, a mixture of two types of strain cultures, or a complex of two types of strains in an amount of 0.001 wt% to 80 wt%, for example, 0.01 wt% to 60 wt%, 0.01 wt% to 40 wt%, 0.01 wt% to 30 wt%, 0.01 wt% to 20 wt%, 0.01 wt% to 10 wt%, 0.01 wt% to 5 wt%, 0.05 wt% to 60 wt%, 0.05 wt% to 40 wt%, 0.05 wt% to 30 wt%, 0.05 wt% to 20 wt%, 0.05 wt% to 10 wt%, 0.05 wt% to 5 wt%, 0.1 wt% to 60 wt%, 0.1 wt% to 40 wt%, 0.1 wt% to 30 wt%, 0.1 wt% to 20 wt%, 0.1 wt% to 10 wt%, or 0.1 wt% to 5 wt%, with respect to the total weight of the composition.

As used herein, the term "included as an active ingredient" means that the strain, the lysate or the culture solution thereof, or the extract of the culture solution of the disclosure is added enough to have the above described effects and means that a formulation is made in various forms by adding various ingredients as sub-ingredients for drug delivery and stabilization.

The composition may be in a liquid state or a dry state. In an embodiment, the composition may be in the form of a dry powder.

As a drying method of preparing the composition in a dry state, a method generally used in the art may be used and is not particularly limited. Non-limiting examples of the drying method include air drying method, a natural drying method, a spray drying method, a freeze drying method, and the like. These methods may be used alone, or at least two methods thereof may be used together.

The composition may include an effective amount of an additive sufficient to reduce the deterioration of the strain, the lysate or the culture solution thereof, or the extract of the culture solution. The additive may be, for example, a binder, but is not limited thereto.

The composition may further include a cosmetically, pharmaceutically, or sitologically acceptable carrier. The composition may be formulated together with a carrier and provided as cosmetics, drugs, food additives, or the like.

The composition may be a cosmetic composition.

In addition to active ingredients provided in the present specification, the cosmetic composition may further include ingredients commonly used in cosmetic compositions, functional additives, or the like. For example, the cosmetic composition may include a carrier and adjuvants such as an antioxidant, a stabilizer, a solubilizer, a surfactant, a dispersant, a preservative, a vitamin, a pigment, and a fragrance.

The cosmetic composition is not particularly limited to a specific formulation, and a formulation may be appropriately selected according to the purpose. The cosmetic composition may have, for example, a solubilized formulation, an emulsion formulation, or a dispersion formulation. The cosmetic composition may have, for example, a cosmetic formulation of a softening lotion, a nourishing lotion, a massage cream, a nourishing cream, an essence, a pack, a gel, an ampoule, or a skin patch type.

The cosmetic composition may be a formulation usable for a hair or a scalp. The cosmetic composition may be, for example, a formulation of a hair tonic, a hair conditioner, a hair essence, a hair lotion, a hair nourishing lotion, a hair shampoo, a hair conditioner, a hair treatment, a hair cream, a hair nourishing cream, a hair moisture cream, a hair massage cream, hair wax, a hair aerosol, a hair pack, a hair nourishing pack, a hair soap, a hair cleansing foam, hair oil, a hair drying preparation, a hair preservation treatment, a hair dye, a hair waving preparation, a hair color-removing preparation, a hair gel, a hair glaze, a hair dressinger, a hair lacquer, a hair moisturizer, a hair mousse, or a hair spray. Specifically, the cosmetic composition may be a formulation of a shampoo, a cleanser, a conditioner, a treatment, a hair pack, a rinse, a scalp pack, a tonic, an essence, a lotion, a cream, oil, a mist, a spray, or a hair gel.

The composition may be a composition for an external preparation for skin.

In the present specification, the external preparation for skin may be a cream, a gel, an ointment, a skin emulsifier, a skin suspension, a transdermal delivery patch, a drug-containing bandage, a lotion, or a combination thereof. The external preparation for skin may be appropriately mixed with an ingredient commonly used in external preparations for skin such as cosmetics or medicines as needed, wherein examples of the ingredient include an aqueous ingredient, an oil-based ingredient, a powder ingredient, alcohols, a moisturizer, a thickener, an ultraviolet absorber, a whitening agent, a preservative, an antioxidant, a surfactant, a fragrance, a colorant, various skin nutrients, or a combination thereof. The external preparation for skin may also be appropriately mixed with metal sequestrants such as disodium edetate, trisodium edetate, sodium citrate, sodium polyphosphate, sodium metaphosphate, and gluconic acid; medicines such as caffeine, tannin, verapamil, a licorice extract, glablidin, a hot water extract of calines from fruit, various herbal medicines, tocopherol acetate, glycyrrhizic acid, tranexamic acid, and a derivative or salt thereof; vitamin C, magnesium ascorbyl phosphate, ascorbate glucoside, arbutin, and kojic acid; sugars such as glucose, fructose, and trehalose.

The composition may be a pharmaceutical composition.

The pharmaceutical composition may additionally include a pharmaceutically acceptable diluent or carrier. The diluent may be lactose, corn starch, soybean oil, microcrystalline cellulose, mannitol, or a combination thereof. The carrier may be an excipient, a disintegrant, a binder, a lubricant, or a combination thereof. The excipient may be microcrystalline cellulose, lactose, low-substituted hydroxycellulose, or a combination thereof. The disintegrant may be calcium carboxymethylcellulose, sodium starch glycolate, calcium monohydrogen phosphate anhydride, or a combination thereof. The binder may be polyvinylpyrrolidone, low-substituted hydroxypropylcellulose, hydroxypropylcellulose, or a combination thereof. The lubricant may be magnesium stearate, silicon dioxide, talc, or a combination thereof.

The pharmaceutical composition may be formulated in an oral or parenteral formulation. The oral formulation may include a granule, a powder, a solution, a tablet, a capsule, a dry syrup, or the like. The parenteral formulation may include a parenteral injection, an ointment, or the like.

The composition may be a health functional food composition. In this case, the composition may be formulated in the form of typical health functional food known in the art.

For the health functional food composition, the strain, the lysate or the culture solution thereof, or the extract of the culture solution may be used alone, or the strain, the lysate or the culture solution thereof, or the extract of the culture solution may be used together with other foods or food ingredients and may be appropriately used according to typical methods. A mixing amount of the active ingredient may be appropriately determined according to the purpose of use (prevention, health, or therapeutic treatment). There is no particular limitation on types of the health functional food. Among the types of health functional foods, a beverage composition may include various flavoring agents or natural carbohydrates as additional ingredients like typical beverages. The natural carbohydrate includes monosaccharide such as glucose or fructose, disaccharide such as maltose or sucrose, polysaccharide such as dextrin or cyclodextrin; and sugar alcohol such as xylitol, sorbitol, or erythritol. A sweeting agent may include a natural sweeting agent such as thaumatin or a stevia extract or a synthetic sweeting agent as saccharin, aspartame, or the like. The health food composition may also include a nutritional supplement, a vitamin, an electrolyte, a flavoring agent, a colorant, pectic acid or a salt thereof, alginic acid or a salt thereof, organic acid, a protective colloidal thickener, a pH adjuster, a stabilizer, a preservative, glycerin, alcohol, a carbonating agent used in carbonated drinks, or a combination thereof. The health functional food composition may also include fruit flesh for preparing natural fruit juices, fruit juice beverages, and vegetable beverages, and a combination thereof.

Another aspect provides a method of preventing, improving, or treating a condition of a subject, the method including treating or administering an effective amount of the composition to a subject in need thereof.

The condition of the subject may be a hair or scalp condition. Specifically, the condition of the subject may be a condition related to alopecia or a condition related to scalp inflammation.

As used herein, the terms "administering," "introducing," and "implanting" may be used interchangeably and may refer to placement of the composition according to an embodiment into a subject through a method or route that leads to at least partial localization of the composition according to an embodiment to a desired site.

The administering may be performed through methods known in the art. The administrating may be performed by directly administering the compound to the subject through any method using a route such as intravenous, intramuscular, oral, transdermal, mucosal, intranasal, intratracheal, or subcutaneous administration. The administrating may be performed systemically or topically.

The subject may be a mammal, for example, a human, a cow, a horse, a pig, a dog, a sheep, a goat, a mouse, a rat, or a cat. The subject may be a subject in need of improvement in hair or scalp condition, for example, a subject in need of hair growth promotion, alopecia prevention, hair root strengthening, scalp lipid suppression, dandruff prevention, or scalp inflammation suppression effects.

The composition according to an embodiment may be administrated in an amount of 0.1 mg to 1,000 mg, for example, 0.1 mg to 500 mg, 0.1 mg to 100 mg, 0.1 mg to 50 mg, 0.1 mg to 25 mg, 1 mg to 1 mg to 1,000 mg, 1 mg to 500 mg, 1 mg to 100 mg, 1 mg to 50 mg, 1 mg to 25 mg, 5 mg to 1,000 mg, 5 mg to 500 mg, 5 mg to 100 mg, 5 mg to 50 mg, 5 mg to 25 mg, 10 mg to 1,000 mg, 10 mg to 500 mg, 10 mg to 100 mg, 10 mg to 50 mg, or 10 mg to 25 mg, per subject per day. However, a dosage may be prescribed in various ways according to factors such as a formulation method, an administration method, patient's age, weight, sex, pathological condition, and diet, a duration of administration, a route of administration, an excretion rate, and response sensitivity. Those skilled in the art may appropriately adjust the dosage considering the factors. Administration frequency may be once or twice or more within a clinically allowable range of side effects, and administration may be given to one site or two or more sites and may be performed every day or at every 2 or 5 days for a total duration of 1 day to 30 days for each treatment. If required, the same treatment may be repeated after an appropriate time. For animals other than humans, a dosage that is the same as that of per kg in a human, or a dosage that is determined by, for example, conversion based on a volume ratio (for example, an average value) of organs (for example, heart) of a target animal and a human, may be administered.

### Advantageous Effects

A novel *Paracoccus denitrificans* strain according to an aspect may have effects of promoting hair growth, preventing alopecia, and strengthening a hair root and thus may be variously applied to improve, prevent, or treat a hair or scalp condition.

In addition, when the *P. denitrificans* strain is used in combination with a *Staphylococcus epidermidis* strain, a synergistic effect of improving a hair or scalp condition may be exhibited.

### Description of Drawings

FIG. 1 is a diagram illustrating a hair structure and a dermal papilla cell.
FIG. 2 shows results of confirming the efficacy of increasing the proliferation ability of dermal papilla cells by each test material.
FIG. 3 is a graph showing a level of expression of a vascular endothelial growth factor (VEGF) gene in dermal papilla cells by each test material.
FIG. 4 is a graph showing a level of expression of a fibroblast growth factor 7 (FGF7) gene in dermal papilla cells by each test material.

### Best Mode

### Mode for Invention

Hereinafter, the disclosure will be described in more detail through examples. However, these examples are for describing the disclosure in more detail, and the scope of the disclosure is not limited to these examples.

### Example 1. Isolation and identification of strain

A scalp of a woman in her 20s with a healthy scalp was washed with sterile distilled water to obtain a sample. The sample was inoculated into an R2A (Reasoner's 2A) liquid medium or solid medium (manufactured by Difco Laboratories Inc.,). After the inoculation, the sample was cultured in an incubator at a temperature of 28 °C for 48 hours, and then formed 100 colonies were purely isolated and cultured to be re-cultured in an incubator at a temperature 28 °C for 48 hours. The cultured colonies were identified by 16S rRNA sequence. In this case, used primers were designed to be amplified by reacting with only bacteria. A polymerase chain reaction (PCR) was performed by 30 cycles under conditions of 95 °C for 1 minute, at 55 °C for 1 minute, and 75 °C for 1 minute and 30 seconds. Finally, the amplified products were maintained at a temperature of 72 °C for 8 minutes and then stored at a temperature 4 °C. After the PCR reaction was completed, DNA sequences of the isolated and cultured species were determined by using ABI-3730XL (manufactured by Applied Biosystems, Inc. (ABI), USA).

When the nucleotide sequence of 16S rRNA determined among isolated and cultured microbial colonies was analyzed by being compared with other strains registered through a BLAST program available on the website of US National Center for Biotechnology Information (NCBI), only novel species with a homology of 97 % or less were selected, and among the novel species, a novel strain of microorganism *P*. *denitrificans* (hereinafter referred to as "SC-1") with a homology of 98 % or less was selected. The selected SC-1 strain was deposited in the Korean Culture Center of Microorganisms on May 28, 2021 with the deposit number KCCM12994P. The SC-1 strain has a 16S rRNA sequence of SEQ ID NO: 1 (complementary DNA).

Meanwhile, *S. epidermidis* (hereinafter referred to as "CICARIA"") was selected in the same manner and deposited under the Korean Culture Center of Microorganisms on June 11, 2019 with the deposit number KCCM12559P. The CICARIA strain has a 16S rRNA sequence of SEQ ID NO: 2 (complementary DNA).

### Example 2. Preparation of SC-1 strain culture solution

The SC-1 strain of Example 1 was inoculated into an R2A (Reasoner's 2A) liquid medium (manufactured by Difco Laboratories Inc.,) and cultured at a temperature of 30 °C for 48 hours, and then the strain was removed with a 0.45 µm filter to prepare an SC-1 strain culture solution.

### Example 3. Preparation of CICARIA strain culture solution

The CICARIA strain of Example 1 was inoculated into an R2A (Reasoner's 2A) liquid medium (manufactured by Difco Laboratories Inc.,) and cultured at a temperature of 30 °C for 48 hours, and then the strain was removed with a 0.45 µm filter to prepare a CICARIA strain culture solution.

### Example 4. Preparation of mixture of SC-1 strain culture solution and CICARIA strain culture solution

The SC-1 strain culture solution of Example 2 and the CICARIA strain culture solution of Example 3 were mixed at a ratio of 1:1 to prepare a strain culture solution mixture (named "CICARIA+SC-1").

### Example 5. Preparation of composite including SC-1 strain culture solution, CICARIA strain culture solution, and yeast extract

The SC-1 strain culture solution of Example 2, the CICARIA strain culture solution of Example 3, and a yeast extract were mixed at a ratio of 1:1:1 to prepare a composite (named "Scalpiome").

### Experimental Example 1. Evaluation of efficacy of increasing proliferation ability of HFDPCs

In order to evaluate whether the strain culture solutions, the mixture, or the composite of Examples 2 to 5 significantly induce the proliferation of dermal papilla cells, 3-(4,5-dimethylthiazol-2-yl)-2,5-diphenyl-2H-tetrazolium bromide (MTT) analysis was performed under the following conditions.

First, HFDPCs were distributed on a 96-well plate at a concentration of 2×10⁴ cells/well and then cultured for 24 hours in an incubator under conditions of a temperature of 37 °C and 5 % CO₂. Next, after a medium was replaced with a medium not including anything else, the culture solution (SC-1) of Example 2, the culture solution (CICARIA) of Example 3, the culture solution mixture (CICARIA+SC-1) of Example 4, the composite (Scalpiome) of Example 5, or a yeast extract was added in an amount of 0.1 % (w/w) and further cultured for 24 hours. A group, to which 10 ppm of minoxidil (MXD having a chemical name of 6-piperidin-1-ylpyrimidine-2,4-diamine 3-oxide) or 2.5 mM of ascorbic acid 2-phosphate (AS2P) was added instead of a strain culture solution, was set as a control group, wherein the MXD or the AS2P was known as a hair growth promoter. In addition, a group (medium control group), to which a medium not including a strain culture solution was added, was set as a negative control group, and in order to confirm the efficacy of a strain culture solution, a group, to which a culture solution was not added, was also set as a control group. After 24 hours, a medium was removed from each well, a washing process was performed once by using Dulbecco's phosphate buffered saline (DPBS), a medium was replaced with a medium including 500 µl of 0.5 mg/mL MTT (manufactured by Sigma-aldrich, St. Louis, USA) for each well, and a reaction was performed at a temperature of 37 °C for 4 hours. Afterwards, absorbance was measured at a wavelength of 570 nm by using Victor 3 (manufactured by Perkin-Elmer Corporation). A survival rate of cells was expressed as a percentage of a measured average absorbance value, and through the survival rate, the proliferative ability of HFDPCs could be confirmed.

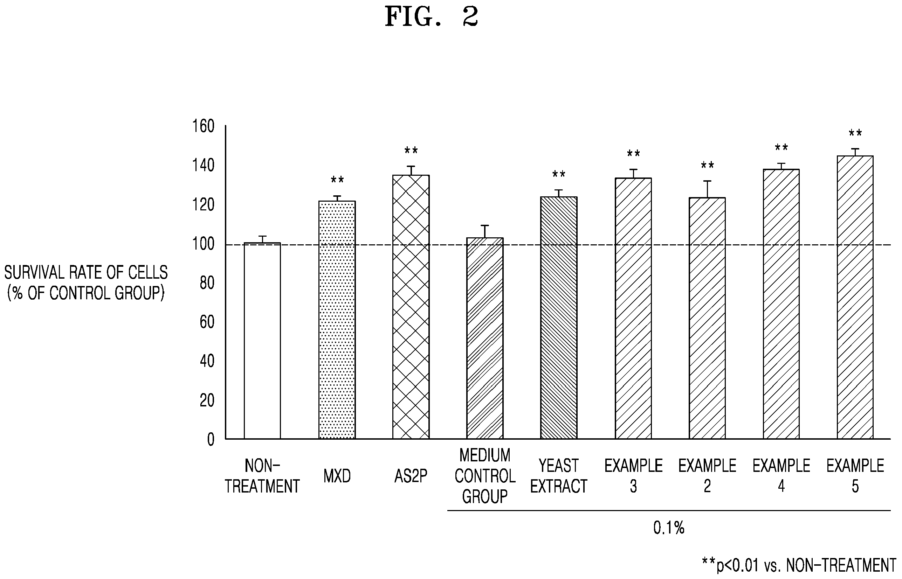

FIG. 2 shows results of confirming the efficacy of increasing the proliferation ability of dermal papilla cells.

As shown in FIG. 2, it could be confirmed that the SC-1 strain culture solution of Example 2, the CICARIA strain culture solution of Example 3, the strain culture solution mixture of Example 4, and the composite of Example 5 all had an effect on the proliferation of dermal papilla cells. In particular, it could be confirmed that the composite of Example 5 had a synergistic effect on the proliferation of dermal papilla cells.

Therefore, it was confirmed that a SC-1 strain, a combination of a SC-1 strain and a CICARIA strain, or a combination of a SC-1 strain, a CICARIA strain, and a yeast extract could be used to strengthen a hair root, prevent alopecia, and promote hair growth.

### Experimental Example 2. Evaluation of efficacy of increasing expression of growth factors (VEGF and FGF7) in HFDPCs

In order to evaluate whether the strain culture solutions, the mixture, or the composite of Examples 2 to 5 could increase the expression of growth factors such as a VEGF and a FGF7 which induced hair growth and maintained scalp health, a quantitative real-time polymerase chain reaction (qRT-PCR) was performed.

Specifically, HFDPCs were distributed on a 6-well plate in an amount of at 3×10⁵ cells/well and then cultured for 24 hours under cell culture conditions. After 24 hours, a medium was discarded, washing was performed by using phosphate buffered saline (PBS), and then cells were starved by using a medium not including fetal bovine serum (FBS). The next day, the medium was replaced with a medium including the culture solution of Example 2 (SC-1), the culture solution of Example 3 (CICARIA), the culture solution mixture of Example 4 (CICARIA+SC-1), the composite (Scalpiome) of Example 5, or a yeast extract in an amount of 0.1 % (w/w), and then culture was additionally performed for additional 24 hours. A group, to which 10 ppm of MXD or 2.5 mM of AS2P was added, was set as a positive control group, a group (medium control group), to which a medium not including a strain culture solution was added, was set to a negative control, and a group, to which a culture solution was not added, was set as a control group. Afterwards, RNA was isolated from cells by using the RNeasy Mini Kit (manufactured by QIAGEN GmbH) and quantified at a wavelength of 260 nm by using nanodrops, and cDNA was synthesized in an amplifier (C1000 thermal cycler manufactured by Bio-Rad, USA) by using 2 µg of each RNA. By performing a qRT-PCR in a real-time PCR machine by using a mixture obtained by adding a primer for VEGF or FGF7 and SYBR Green supermis (manufactured by ABI, USA) as a cyanine dye to the synthesized cDNA, finally, a level of expression of VEGF and FGF7 genes were evaluated. The sequences of used primers and reaction conditions are shown in Table 1 below. An expression level of a gene was finally analyzed by correcting a β-actin gene.

**[Table 1]**

| Primer name | Direction | Sequence | SEQ ID No. | Reaction conditions |
|---|---|---|---|---|
| VEGF | F | 5'-GTGCCCACTGAGGAGTTCAAC- 3' | 3 | After polymerase activation at temperature of 94 °C for 5 minutes, polymerization |
| | R | 5'-CCCTATGTGCTGGCCTTGAT-3' | 4 | |
| FGF7 | F | 5'-TCCTGCCAACTTTGCTCTACA-3' | 5 | |
| | R | 5'-CAGGGCTGGAACAGTTCACAT- 3' | 6 | reaction was performed by 40 cycles under conditions of temperature of 95 °C for 30 seconds, temperature of 60 °C for 30 seconds, and temperature of 72 °C for 30 seconds |
| β-actin | F | 5'-GGCCATCTCTTGCTCGAAGT-3' | 7 | |
| | R | 5'-GAGACCTTCAACACCCCAGC-3' | 8 | |

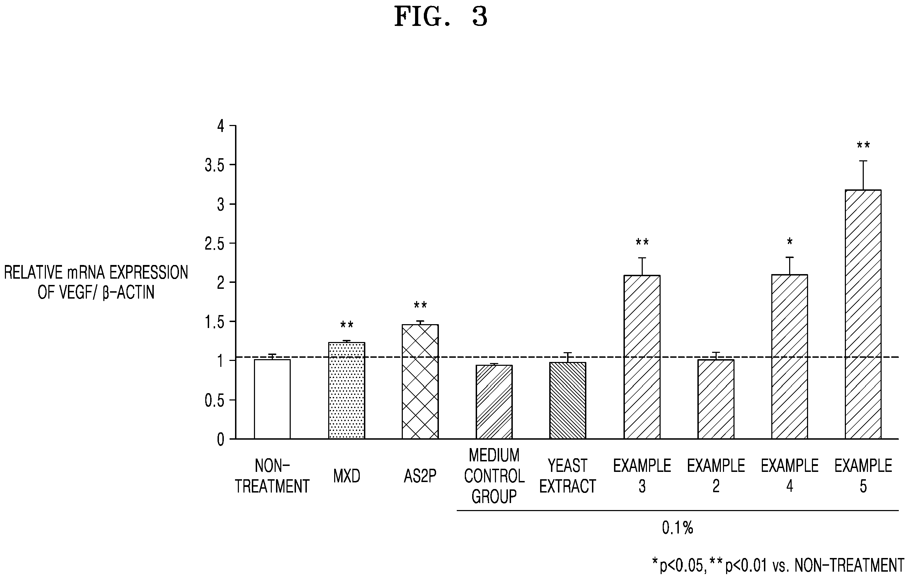

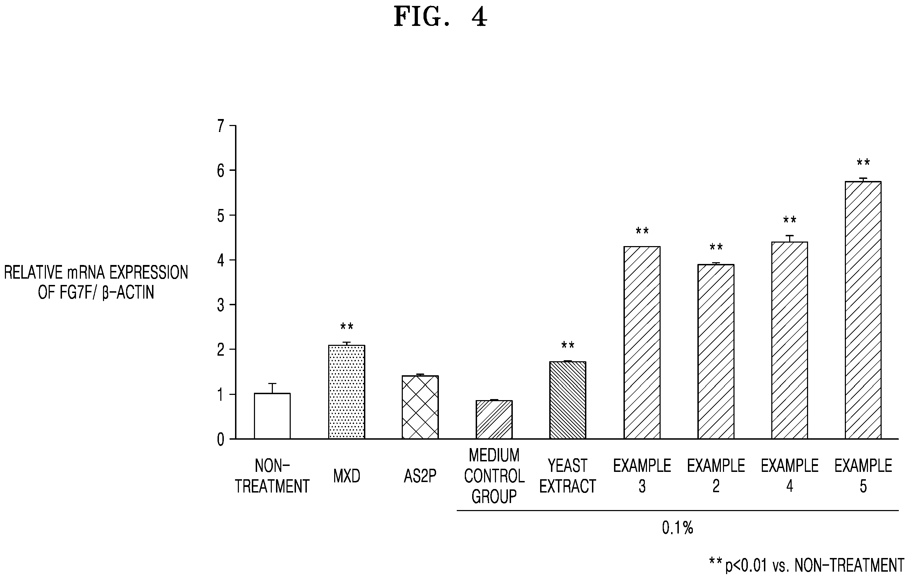

FIG. 3 is a graph showing a level of expression of a VEGF gene in dermal papilla cells by each test material. FIG. 4 is a graph showing a level of expression of a FGF7 in dermal papilla cells by each test material. As shown in FIGS. 3 and 4, it could be confirmed that the SC-1 strain culture solution of Example 2 had an excellent effect of increasing the expression of an FGF7. It could be confirmed that the CICARIA strain culture solution of Example 3, the strain culture mixture of Example 4, and the composite of Example 5 all had an excellent effect of increasing the expression of a VEGF and a FGF7. In particular, it could be confirmed that the composite of Example 5 had a synergistic effect of increasing the expression of a VEGF and an FGF7.

Therefore, it was confirmed that a SC-1 strain, a combination of a SC-1 strain and a CICARIA strain, or a combination of a SC-1 strain, a CICARIA strain, and a yeast extract could be used for strengthening a hair root, preventing alopecia, promoting hair growth, and improving scalp health.

The above description is merely an exemplary description of the technical spirit of the disclosure, and those skilled in the art to which the disclosure pertains may make various modifications and variations without departing from the essential characteristics of the disclosure.

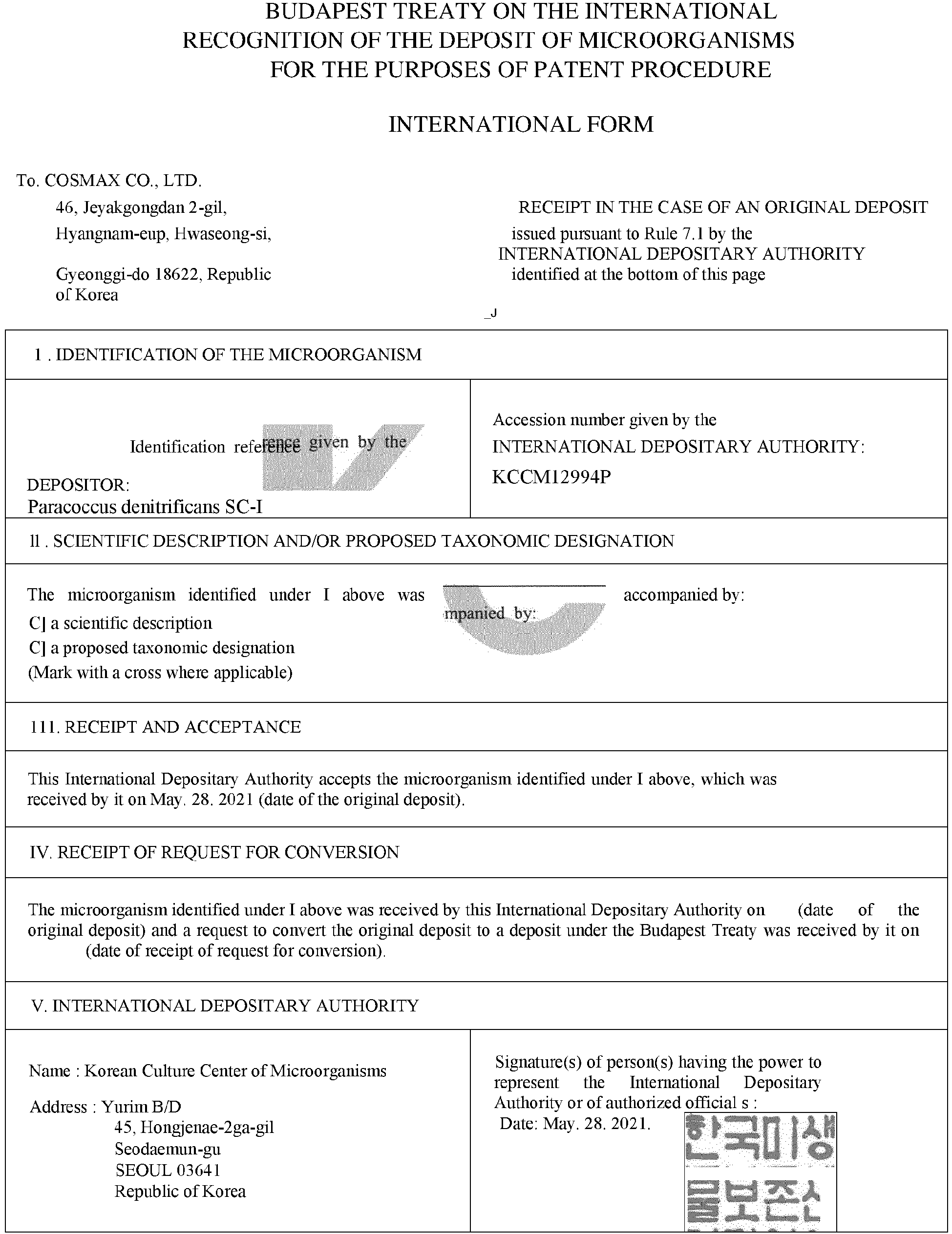
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. COSMAX CO., LTD.
46, Jeyakgongdan 2-gil,
Hyangnam-eup, Hwaseong-si,
Gyeonggi-do 18622, Republic
of Korea

RECEIPT IN THE CASE OF AN ORIGINAL DEPOSIT
issued pursuant to Rule 7.1 by the
INTERNATIONAL DEPOSITARY AUTHORITY
identified at the bottom of this page

_J

1 . IDENTIFICATION OF THE MICROORGANISM
Identification reference given by the
DEPOSITOR:
Paracoccus denitrificans SC-I | Accession number given by the
INTERNATIONAL DEPOSITARY AUTHORITY:
KCCM12994P
Il . SCIENTIFIC DESCRIPTION AND/OR PROPOSED TAXONOMIC DESIGNATION
The microorganism identified under I above was accompanied by:
mpanied by:
C] a scientific description
C] a proposed taxonomic designation
(Mark with a cross where applicable)
111. RECEIPT AND ACCEPTANCE
This International Depositary Authority accepts the microorganism identified under I above, which was
received by it on May. 28. 2021 (date of the original deposit).
IV. RECEIPT OF REQUEST FOR CONVERSION
The microorganism identified under I above was received by this International Depositary Authority on (date of the
original deposit) and a request to convert the original deposit to a deposit under the Budapest Treaty was received by it on
(date of receipt of request for conversion).
V. INTERNATIONAL DEPOSITARY AUTHORITY
Name : Korean Culture Center of Microorganisms
Address : Yurim B/D
45, Hongjenae-2ga-gil
Seodaemun-gu
SEOUL 03641
Republic of Korea | Signature(s) of person(s) having the power to
represent the International Depositary
Authority or of authorized official s :
Date: May. 28. 2021.
한국미생
물보존[?]

Where Rule 6.4(d) applies, such date is the date on which the status of int | ti | uthority was
acquired.
Form BP/4 (sole page)
KOREAN CULTURE CEhffER OF MICROORGANISMS
Yurr-n Bldg.. 45. Hongjenae 2ga-gll, Seodaemun-gu. Seoul. 03641. Korea. Te2: 82-2-391 -0950. 396-0950 Fax; 82-2-392-2859

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. COSMAX CO., LTD.
46, Jeyakgongdan 2-gil,
Hyangnam-eup, Hwaseong-si,
Gyeonggi-do, 18622,
Republic of Korea

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Staphylococcus epidermidis CICARIA | Accession number given by the
INTERNATIONAL DEPOSITARY AUTHORITY:
KCCM12559P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on June. 11. 2019 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: June. 11. 2019.

¹ Where Rule 6.4(d) applies, such date is the date on which the status of international depositary authority was
acquired.

Form BP/4 (sole page)

## Claims

1. A *Paracoccus denitrificans* strain comprising 16S rRNA having a sequence identity of 95 % or more with SEQ ID NO: 1.

2. The strain of claim 1, wherein the strain is deposited under the deposit number KCCM12994P.

3. A culture solution of the strain of claim 1.

4. A cosmetic composition comprising the strain of claim 1, a lysate or a culture solution thereof, or an extract of the culture solution.

5. The cosmetic composition of claim 4, wherein the cosmetic composition is for improving a hair or scalp condition.

6. The cosmetic composition of claim 5, wherein the improving of the hair or scalp condition is at least one of hair growth promotion, alopecia prevention, and hair root strengthening.

7. The cosmetic composition of claim 4, wherein the cosmetic composition proliferates human follicular dermal papilla cells or increases an expression of at least one of a vascular endothelial growth factor (VEGF) and a fibroblast growth factor 7 (FGF7).

8. The cosmetic composition of claim 4, further comprising *Staphylococcus epidermidis* deposited under the deposit number KCCM12559P, a lysate or a culture solution thereof, or an extract of the culture solution.

9. A cosmetic composition for improving a hair or scalp condition, the cosmetic composition comprising a *Paracoccus denitrificans* strain, a lysate or a culture solution thereof, or an extract of the culture solution.

10. The cosmetic composition of claim 9, further comprising *Staphylococcus epidermidis* deposited under the deposit number KCCM12559P, a lysate or a culture solution thereof, or an extract of the culture solution.

11. The cosmetic composition of claim 9 or 10, further comprising a yeast extract.

12. A pharmaceutical composition for promoting hair growth or preventing alopecia, the pharmaceutical composition comprising a *Paracoccus denitrificans* strain, a lysate or a culture solution thereof, or an extract of the culture solution as an active ingredient.

13. The pharmaceutical composition of claim 12, further comprising: at least one of *Staphylococcus epidermidis* deposited under the deposit number KCCM12559P, a lysate or a culture solution thereof, and an extract of the culture solution; and a yeast extract.

14. A health functional food composition for improving a hair or scalp condition, the health functional food composition comprising a *Paracoccus denitrificans* strain, a lysate or a culture solution thereof, or an extract of the culture solution.

15. The health functional food composition of claim 14, further comprising: at least one of *Staphylococcus epidermidis* deposited under the deposit number KCCM12559P, a lysate or a culture solution thereof, and an extract of the culture solution; and a yeast extract.

16. Use of a Paracoccus *denitrificans* strain, a lysate or a culture solution of the strain, or an extract of the culture solution.

17. A method of preventing, improving, or treating alopecia of a subject, the method comprising treating or administering an effective amount of a composition to a subject in need thereof, wherein the composition comprises, as an active ingredient, a *Paracoccus denitrificans* strain, a lysate or a culture solution thereof, or an extract of the culture solution.
